# EUROPÄISCHE PATENTSCHRIFT

(11) **EP 0 811 354 B1**
(45) Veröffentlichungstag und Bekanntmachung des Hinweises auf die Patenterteilung: **05.03.2003**
(21) Anmeldenummer: 97108199.7
(22) Anmeldetag: 21.05.1997
(51) Int. Cl.: A61B 17/22

(54) **Vorrichtung zur Ortung und Zertrümmerung von Konkrementen**
Device for the location and disintegration of concretions in the body
Dispositif de détection et destruction de concrétions dans le corps

(30) Priorität: 07.06.1996 DE 19622919
(43) Veröffentlichungstag der Anmeldung: 10.12.1997
(73) Patentinhaber: Dornier Medizintechnik GmbH, 82334 Wessling (DE)
(72) Erfinder: Überle, Friedrich, Dipl.-Ing. Dr., 82205 Gilching (DE)
(74) Vertreter: Grünecker, Kinkeldey, Stockmair & Schwanhäusser Anwaltssozietät

(56) Entgegenhaltungen:
- EP-A- 0 316 863
- EP-A- 0 369 177
- EP-A- 0 400 196
- EP-A- 0 441 997
- FR-A- 2 587 493

## Beschreibung

Die vorliegende Erfindung betrifft eine Vorrichtung zur Ortung und Zertrümmerung von Konkrementen im Körper von Patienten mit einem Stoßwellengenerator, mit einer Koppelanordnung zur Übertragung der Stoßwellen in den Körper des Patienten und mit einer Ortungsanordnung zur genauen Ermittlung der Lage des Konkrementes.

Derartige Vorrichtungen, auch Lithotripter genannt, sind viefältig bekannt. So beschreibt die DE-OS 3532678 einen derartigen Lithotripter, dessen Stoßwellen über eine den Körper des Patienten direkt berührende oder indirekt berührende Flüssigkeit einer flexibel gestalteten Flüssigkeitsvorlaufstrecke übertragen und dadurch auf den georteten Stein fokussiert werden. Der Patient befindet sich auf einer Liegefläche, die mit einem Fenster versehen ist, unter dem der Stoßwellengenerator mit der flexiblen Flüssigkeitsvorlaufstrecke derart angeordnet ist, daß dessen Flüssigkeit nach außen abgedichtet durch das Fenster die zu beschallende in das Fenster eintauchende Körperfläche des Patienten direkt oder über eine die Vorlaufstrecke abdichtende Membran berührt.

Die DE-OS 4124259 betrifft einen Lithotripter mit einem Schallwellenerzeuger und mit einer mit Koppelflüssigkeit gefüllten Vorlaufstrecke und mit einer Membran, die zwischen Schallerzeuger und Patientenkörper angeordnet ist; die Vorlaufstrecke weist einen ersten nach außen hin abgeschlossenen Raum zwischen Schallwellenerzeuger und Membran sowie einen zweiten offenen und durch den Patientenkörper abschließbaren Raum zwischen Membran und Patientenkörper auf.

Nachteilig bei diesen bekannten Lithotriptern ist, daß die von der Ortungsanordnung zur Feststellung der genauen Lage des im Körper des Patienten befindlichen und zu zertrümmernden Konkrementes ausgesandten der Ortung dienenden Schallwellen, die üblicherweise koaxial zur Achse der später abgestrahlten Stoßwellen auf den Patientenkörper gerichtet werden, die zwischen der Koppelanordnung und dem Patienten vorhandene, die Koppelflüssigkeit zurückhaltende Membran sowohl beim Eindringen in den Körper als auch bei der Reflexion durch das Konkrement durchdringen müssen, wodurch die Bildqualität der Ortungsanordnung beeinträchtigt wird.

Aus der EP A 441 997 ist ein Stoßwellengenerator zur Erzeugung von Stoßwellen in einem akustischen Ausweitungsmedium bekannt, mit einem Ultraschall-Applikator, mittels dessen für Ortungszwecke Ultraschallwellen in das akustische Ausbreitungsmedium einleitbar sind und der wenigstens einen Ultraschall-Transducer aufweist; wenigstens einem Teil eines im Ausbreitungsweg der Stoßwellen befindlichen Bereiches des Ultraschall-Applikators ist ein Stoff vorgelagert, dessen akustische Impedanz erheblich von der des akustischen Ausbreitungsmediums abweicht. Bei diesem bekannten Stoßwellengenerator besteht der Ultraschall-Applikator aus einem hohlzylindrischen rohrförmigen, die Ultraschall-Transducer aufnehmenden Gehäuse, das an seinem, dem Patienten zugewandten Ende ein kuppelförmiges Verschlußteil aufweist, in dem das Schallaustrittsfenster für die Ortung vorgesehen ist. Der Applikator ragt dabei in das Koppelkissen hinein, wird jedoch auch hierbei in der den Körper des Patienten zugewandten, die Koppelflüssigkeit umschließenden Membran allseitig umgeben, so daß auch die vom Applikator ausgesandten Ortungswellen in Richtung zum Konkrement diese Membran zweimal passieren müssen.

Aufgabe der vorliegenden Erfindung ist es, die Bildqualität der Ortungsanordnung zur genauen Festlegung der Lage des Konkrementes durch einen einfachen Aufbau deutlich zu verbessern.

Die Lösung dieser Aufgabe erfolgt mit den dem kennzeichnenden Teil des Anspruchs 1 angegebenen Merkmalen; vorteilhafte Ausgestaltungen sind in den Unteransprüchen beschrieben. Die Merkmale des Oberbegriffs des Anspruchs 1 sind aus Dokument EP-A-0 316 863 bekannt.

Durch die erfindungsgemäße Ausgestaltung der Koppelanordnung in Form eines ringförmigen Koppelkissens mit einem zentralen Durchlaß, welcher die Ortungsanordnung im wesentlich symmetrisch umgibt, wobei letztere sowohl in Axialrichtung verschiebbar als auch um die Längsachse verdrehbar in den Durchlaß im Koppelkissen eingesetzt wird und wobei aufgrund des mit der Ortungsanordnung verbundenen und im Durchlaß mündenden Flüssigkeitseinlasses undauslasses können die von der Ortungsanordnung ausgesandten Schallwellen ungestört und ungehindert durch eine Membran in den Körper eindringen und vom Konkrement reflektiert werden, so daß ein klareres exakteres Bild der Lage des Konkrementes erhalten wird. Der Raum zwischen der dem Patienten zugewandten Oberfläche der Ortungsanordnung und der Körperoberfläche des Patienten wird durch den Flüssigkeitszulauf und- ablauf mit einer geeigneten Koppelflüssigkeit gefüllt, die nicht mit der Koppelflüssigkeit im Koppelkissen in Verbindung steht. Dies bringt den Vorteil mit sich, daß nur die geringe zwischen Körperoberfläche des Patienten und Ortungsanordnung befindliche Koppelflüssigkeit bei jedem Patientenwechsel auszutauschen ist.

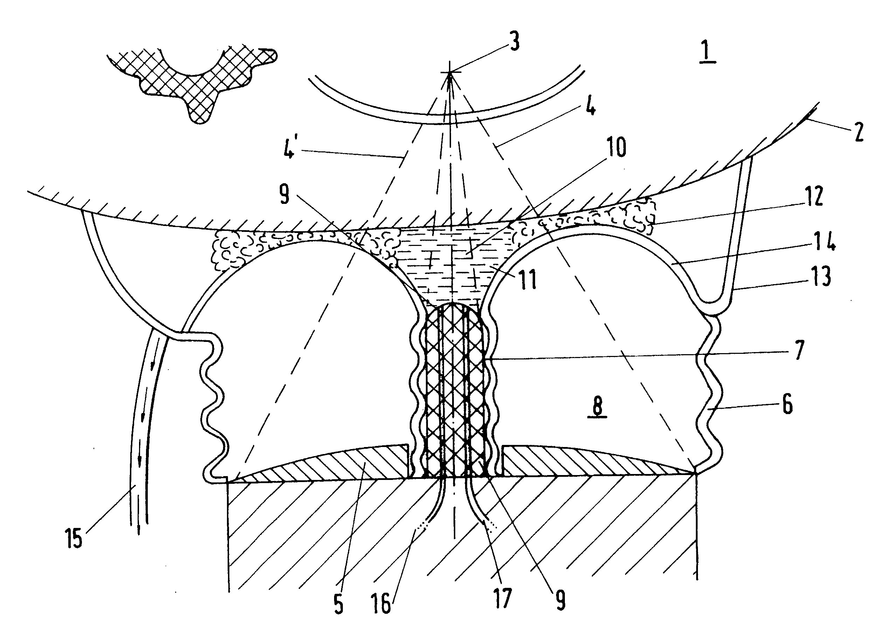

Im folgenden wird die Erfindung anhand der Zeichnung näher erläutert, in der ein vorteilhaftes Ausführungsbeispiel dargestellt ist.

Die einzige Figur zeigt einen Schnitt durch die wesentlichen Teile einer erfindungsgemäß ausgestalteten Vorrichtung zur Ortung und zur Zertrümmerung von Konkrementen im Körper eines Patienten.

In der einzigen Figur ist mit 1 ein Teil des Patientenkörpers bezeichnet und mit 2 die Oberfläche, d. h. die Haut. Im Körper des Patienten 1 befindet sich ein zu zertrümmendes Konkrement 3, z. B. ein Nierenstein. Der nur teilweise dargestellte Lithotripter besteht aus einem (nicht dargestellten) Stoßwellengenerator, einer Koppelanordnung 6 zur Übertragung der Stoßwellen, deren Randstrahlen gestrichelt mit 4 und 4' bezeichnet sind und aus einer Ortungsanordnung 9 zur genauen Ermittlung der Lage des Konkrementes 3 im Körper des Patienten 1. Die vom Stoßwellengenerator erzeugten parallel ausgesandten Stoßwellen werden hierbei durch eine fokussierende an und für sich bekannte Anordnung 5 in Richtung zum Konkrement 3 hin gebündelt.

Zur genauen Ermittelung der Lage des Konkrementes 3 und damit zur exakten Fokussierung der Stoßwellen dient die schematisch dargestellte Ortungsanordnung 9, von der Ultraschallwellen möglichst ungestört in den Körper des Patienten eingestrahlt werden müssen. Um diesen Zweck gemäß der vorliegenden Erfindung zu erreichen, ist nun die herkömmliche Koppelanordnung 6 in Form eines ringförmigen elastisch verformbaren Koppelkissens ausgeführt, das einen zentralen Durchlaß 7 aufweist. Das Koppelkissen 6, das aus einem flexiblen ringförmigen Balg 6 und einer, diesen Ringraum in Richtung zum Patienten 1 hin abschließenden elastisch verformbaren ringförmigen Membran 14 besteht, ist mit einer geeigneten Koppelflüssigkeit 8 zum Einleiten der Stoßwellen 8 gefüllt und in flüssigkeitdichter Weise mit der Fokussieranordnung 5 des Stoßwellengenerators verbunden.

An der Außenseite des ringförmigen Koppelkissens 6 ist vorteilhafterweise eine elastische umlaufende Auffangrinne 13 vorgesehen, die derart ausgestaltet ist, daß ihr freier Rand bei Anliegen des Koppelkissens 6 am Körper des Patienten 1 ebenfalls an diesem anliegt, so daß der durch die Membran 14 des Koppelkissens und die Auffangrinne 13 gebildete Ringraum ebenfalls mit einer Koppelflüssigkeit gefüllt werden kann. Gegebenenfalls ist die Auffangrinne 13 mit einem mit 15 angedeuteten Zulauf und/oder Ablauf versehen.

Zwischen der elastischen Membran 14 des Koppelkissens und der Haut 2 des Patienten kann eine kreisringförmige Gelscheibe 12 zur Verbesserung der Übertragung der Stoßwellen und zur Abdichtung des zwischen Ortungsanordnung 9 und Körperoberfläche 2 vorhandenen, den Durchlaß verlängernden Raumes 10 vorgesehen sein, wobei der Raum 10 mit einer geeigneten, die Einleitung der von Ortungsanordnung 9 in den Patientenkörper dienenden Flüssigkeit 11 gefüllt sein kann.

Die Ortungsanordnung 9 wird zu diesem Zweck vorteilhafterweise mit einem mit 16 angedeuteten Zulauf und einem mit 17 angedeuteten Ablauf versehen zum Einfüllen der Koppelflüssigkeit 11 in den Raum 10.

Es ist klar, daß die im Koppelkissen 6 vorhandene Koppelflüssigkeit 8 für die Einleitung der Stoßwellen vom Stoßwellengenerator bei Patientenwechsel nicht ausgetauscht werden muß, da diese Flüssigkeit mit dem Patienten selbst nicht in Berührung steht, sondern durch die Membran 14 und ggf. durch die Gelscheibe 12 von diesem getrennt ist. Nur die geringe, im Raum 10 vorhandene Flüssigkeitsmenge 11 wird bei jedem Patientenwechsel bzw. nach Beendigung der Behandlung entfernt.

Vorzugsweise sind Zulauf und Ablauf für den Raum 10 mit einer Flüssigkeitspumpe versehen; auch Zulauf und Ablauf für die Auffangrinne 13 können mit einer geeigneten Pumpe versehen sein. Besonders günstig ist es, wenn die im Durchlaß 7 im ringförmigen Koppelkissen 6 angeordnete Ortungsanordnung 9 sowohl in Axialrichtung verschiebbar als auch um ihre Längsachse durch eine (nicht dargestellte) Betätigungsvorrichtung verdrehbar ist. Eine geeignete Betätigungsvorrichtung zur axialen Verschiebung der Ortungsanordnung 9 kann in kostengünstiger und einfacher weise darin bestehen, daß die Außenwandung des den Durchlaß 7 durchsetzenden Teils der Ortungsanordnung 9 derart ausgestaltet ist, daß bei Druckbeaufschlagung des Koppelkissens 6 die, den Durchlaß 7 umgebende Wandung des Koppelkissens 6 die Ortungsanordnung 9 in Richtung des Patienten 1 bewegt.

Vorteilhafterweise ist die Ortungsanordnung 9 mit einem Art Sensor versehen, der dessen Relativlage zum Koppelkissen und damit zum Patienten 1 feststellt und einer Überwachungsanordnung des Lithotripters meldet.

## Patentansprüche

1. Vorrichtung zur Ortung und Zertrümmerung von Konkrementen im Körper von Patienten, mit einem Stoßwellengenerator, mit einer Koppelanordnung (6) zur Übertragung der Stoßwellen in den Körper des Patienten (1) und mit einer Ortungsanordnung (9) zur genauen Ermittlung der Lage des Konkrementes,
wobei die Koppelanordnung aus einem ringförmigen, mit einem Durchlaß (7) versehenen Koppelkissen (6) besteht, das die Ortungsanordnung (9) umgibt, das in flüssigkeitsdichter Weise mit dem Stoßwellengenerator verbunden ist, das mit einem Flüssigkeitszulauf und -ablauf versehen ist, **dadurch gekennzeichnet, daß** die Ortungsanordnung (9) mit einem Flüssigkeitszulauf (16) und -ablauf (17) versehen ist, die beide in der Nähe des dem Patienten zugewandten Endes der Ortungsanordnung (9) und damit im Raum (10) zwischen Patient und Durchlaß (7) im Koppelkissen münden.

2. Vorrichtung nach Anspruch 1, **dadurch gekennzeichnet, daß** das Koppelkissen (6) an einem ringförmigen, den zentralen Durchlaß umgebenden Gelring (12) anliegt.

3. Vorrichtung nach einem der vorhergehenden Ansprüche, **dadurch gekennzeichnet, daß** die Außenseite des Koppelkissens mit einer elastischen umlaufenden Auffangrinne (13) versehen ist, deren freier Rand dazu dient, am Körper des Patienten anzuliegen.

4. Vorrichtung nach Anspruch 3 **dadurch gekennzeichnet, daß** die Auffangrinne (13) mit einem Flüssigkeitszulauf (15), einem Flüssigkeitsablauf (15) und mit einer Pumpe versehen ist.

5. Vorrichtung nach einem der vorhergehenden Ansprüche, **dadurch gekennzeichnet, daß** die im Durchlaß (7) angeordnete Ortungsanordnung (9) in Axialrichtung verschiebbar und/oder in die Längsachse verdrehbar ist.

6. Vorrichtung nach einem der vorhergehenden Ansprüche, **dadurch gekennzeichnet, daß** die Außenwand der Ortungsanordnung (9), die mit der Innenwand des Koppelkissens im Einsatz in Berührung steht, derart geformt ist, daß die Ortungsanordnung (9) bei Druckbeaufschlagung des Koppelkissens (6) in Richtung Patient bewegt wird.

7. Vorrichtung nach einem der vorhergehenden Ansprüche, **dadurch gekennzeichnet, daß** die Ortungsanordnung (9) mit einem Ortssensor versehen ist.

## Claims

1. Device for the location and lithotripsy of concretions in a patient's body, comprising a shock wave generator with a coupling arrangement (6) for transmitting the shock waves to the patient's body (1) and with a locating arrangement (9) for accurately determining the position of the concretion, wherein the coupling arrangement consists of an annular coupling cushion (6) provided with a passage (7), which cushion surrounds the locating arrangement (9), is connected in a liquid-tight manner to the shock wave generator and is provided with a liquid feed line and discharge line, **characterised in that** the locating arrangement (9) is provided with a liquid feed line (16) and liquid discharge line (17), both of which terminate in the vicinity of the end of the locating arrangement (9) facing the patient and thereby in the space (10) between the patient and the passage (7) in the coupling cushion.

2. Device according to claim 1, **characterised in that** the coupling cushion (6) rests against an annular gel ring (12) surrounding the central passage.

3. Device according to one of the preceding claims, **characterised in that** the outside of the coupling cushion is provided with an elastic circumferential engagement groove (13) whose free end is designed to rest against the patient's body.

4. Device according to claim 3, **characterised in that** the engagement groove (13) is provided with a liquid feed line (15), a liquid discharge line (15) and with a pump.

5. Device according to one of the preceding claims,
**characterised in that** the locating arrangement (9) positioned in the passage (7) can be displaced in the axial direction and/or can be rotated about the longitudinal axis.

6. Device according to one of the preceding claims, **characterised in that** the outer wall of the locating arrangement (9), which is in contact with the inner wall of the coupling cushion during use, is formed in such a way that the locating arrangement (9) is moved in the direction of the patient when the coupling cushion (6) is subjected to pressure.

7. Device according to one of the preceding claims, **characterised in that** the locating arrangement (9) is provided with a locating sensor.

## Revendications

1. Dispositif de repérage et de destruction de concrétions dans le corps d'un patient, comprenant un générateur d'ondes de choc, un dispositif de liaison (6) pour transmettre les ondes de choc vers le corps du patient (1) et un dispositif de repérage (9) pour déterminer avec précision la position de la concrétion, le dispositif de liaison étant formé par un coussin de liaison (6) annulaire, qui est muni d'un passage (7) et entoure le dispositif de repérage (9), qui est relié, de manière étanche aux liquides, avec le générateur d'ondes de choc, qui est muni d'une entrée et d'une sortie de liquide, **caractérisé en ce que** le dispositif de repérage (9) est muni d'une entrée de liquide (16) et d'une sortie de liquide (17), qui débouchent toutes les deux à proximité de l'extrémité du dispositif de repérage (9) orientée vers le patient, et, de ce fait, dans l'espace (10) entre le patient et le passage (7) dans le coussin de liaison.

2. Dispositif selon la revendication 1, **caractérisé en ce que** le coussin de liaison (6) est en appui contre un anneau de gel (12) circulaire entourant le passage central.

3. Dispositif selon une des revendications précédentes, **caractérisé en ce que** le côté extérieur du coussin de liaison est muni d'une rigole de réception (13) périphérique élastique, dont le bord libre est destiné à être mis en appui contre le corps du patient.

4. Dispositif selon la revendication 3,
**caractérisé en ce que** la goulotte de réception (13) est munie d'une entrée de liquide (15), d'une sortie de liquide (15) et d'une pompe.

5. Dispositif selon une des revendications précédentes, **caractérisé en ce que** le dispositif de repérage (9) agencé dans le passage (7) est mobile dans le sens axial et/ou peut être entraîné en rotation autour de l'axe longitudinal.

6. Dispositif selon une des revendications précédentes, **caractérisé en ce que** la paroi extérieure du dispositif de repérage (9), qui, en cours de service, est en contact avec la paroi intérieure du coussin de liaison, est formée de telle sorte que le dispositif de repérage (9) se déplace en direction du patient lorsque le coussin de liaison (6) est sollicité par une pression.

7. Dispositif selon une des revendications précédentes, **caractérisé en ce que** le dispositif de repérage (9) est muni d'un capteur de position.
